(19) Europäisches Patentamt
European Patent Office
Office européen des brevets

(11) Numéro de publication : **0 152 366**
**B1**

(12) # FASCICULE DE BREVET EUROPÉEN

(45) Date de publication du fascicule du brevet : **28.06.89**

(51) Int. Cl.⁴ : **A 61 K 33/24** // (A61K33/24, 31:695)

(21) Numéro de dépôt : **85450004.8**

(22) Date de dépôt : **08.02.85**

(54) **Compositions à usage thérapeutique comprenant des composés organo-siliciés.**

(30) Priorité : **08.02.84 FR 8402120**

(43) Date de publication de la demande :
**21.08.85 Bulletin 85/34**

(45) Mention de la délivrance du brevet :
**28.06.89 Bulletin 89/26**

(84) Etats contractants désignés :
**AT BE CH DE GB IT LI LU NL SE**

(56) Documents cités :
**FR-A- 2 158 068**
**FR-A- 2 160 293**
**FR-A- 2 230 376**
**FR-M- 1 069**
**GB-A- 2 033 915**

(73) Titulaire : **Le Ribault, Loic**
**23, Chemin des Dames**
**F-33260 La-Teste-Du-Buch (FR)**

**Nardou, René**
**32, Allée Emile Pereire**
**F-33120 Arcachon (FR)**

(72) Inventeur : **Le Ribault, Loic**
**23, Chemin des Dames**
**F-33260 La-Teste-Du-Buch (FR)**
Inventeur : **Nardou, René**
**32, Allée Emile Pereire**
**F-33120 Arcachon (FR)**

(74) Mandataire : **Thébault, Jean-Louis**
**Cabinet Thébault S.A. 50 Cours de Verdun**
**F-33000 Bordeaux (FR)**

**EP 0 152 366 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne de nouvelles compositions à usage thérapeutique, notamment utiles pour le traitement de certains dérèglements de la mitose cellulaire et des maladies cardio-vasculaires.

Les composés organo-siliciés sont déjà utilisés en thérapeutique humaine depuis de nombreuses années notamment grâce aux découvertes de N. Duffaut.

Ces composés hydrosolubles et atoxiques traversent aisément l'épiderme et le derme par application locale (Gueyne, Duffaut et Quilichini, Thérapie 1962, 17, 417).

Les nombreuses propriétés thérapeutiques de ces composés organo-siliciés, utilisés seuls, ont été exposées dans plusieurs brevets (en particulier les brevets français 2.158.068, 2.160.293 et 2.230.376).

L'administration de ces composés organo-siliciés se fait, selon ces documents, par voie intramusculaire ou intraveineuse, ou encore par électrophorèse, le principe actif étant alors en solution dans l'eau (solution isotonique), éventuellement additionnée d'un alcool ou d'un polyalcool, tel que le glycérol et/ou d'un sel de sodium d'un acide organique pharmaceutiquement acceptable.

Il avait été démontré, en particulier dans les brevets mentionnés ci-dessus, que certaines substances de nature diverse pouvaient potentialiser et élargir le spectre d'action de ces composés organo-siliciés.

On a maintenant trouvé de façon inattendue qu'on peut réaliser une nouvelle composition à usage thérapeutique en combinant un ou plusieurs composés organo-siliciés avec un ou plusieurs métaux, de préférence sous le forme de sels (chlorure, nitrate, sulfate ou sel d'acide organique par exemple) ou sous la forme d'oxydes ou d'hydroxydes, toujours à titre d'exemple, sans que cela soit limitatif.

Les métaux concernés sont le titane, le zirconium, le hafnium, le vanadium, le chrome, le germanium, le rhodium, l'or, l'iridium, le platine et/ou l'osmium, mais on peut aussi utiliser les dérivés uranylés et les terres rares.

On a également trouvé que les compositions de ce type sont tout particulièrement utiles pour le traitement de certains dérèglements de la mitose cellulaire et des maladies cardio-vasculaires contre lesquelles elles exercent une action spécifique, et que les compositions ainsi réalisées peuvent être administrées simplement par voie transcutanée.

L'invention a donc pour premier objet une composition à usage thérapeutique, notamment utile pour le traitement de dérèglements de la mitose cellulaire et des maladies cardio-vasculaires, caractérisée en ce qu'elle comprend, en solution aqueuse :

(1) au moins un composé organo-silicié répondant à l'une des deux formules :

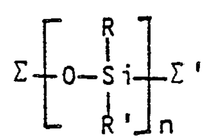

ou

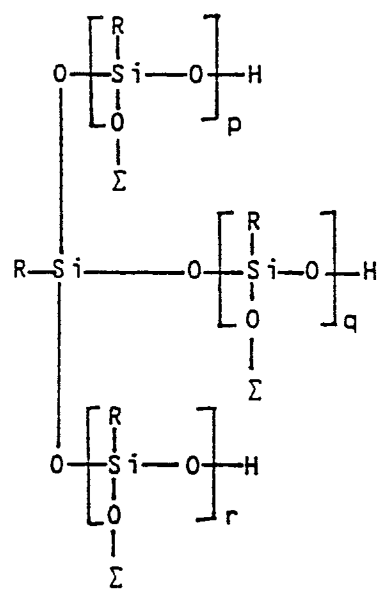

(A) (B)

dans lesquelles :

n est un nombre entier compris entre 1 et 1 000, et de préférence entre 1 et 50 ;

p, q, r sont des nombres entiers compris entre 0 et 1 000, et de préférence entre 0 et 60 ;

R et R' représentent, indépendamment l'un de l'autre, un groupement aliphatique linéaire, ramifié ou cyclique, saturé ou non, hétéro-linéaire ramifié ou non, cyclique saturé ou non, aromatique, hétéroaromatique, arylaliphatique, ou hétéroarylaliphatique, pouvant être également fonctionnel tandis que R peut aussi être OH, OR ou $OSiR_3$ ;

Σ est R, H ou $SiR_3$, où R est tel que précédemment défini ;

Σ' est R et plus particulièrement $CH_3$, OR, OH ou $OSiR_3$, où R est tel que défini ci-dessus, et

(2) au moins un métal choisi parmi le titane, le zirconium, l'hafnium, le vanadium, le germanium, le chrome, le rhodium, l'or, l'iridium, le platine, l'osmium, les dérivés uranylés et les terres rares, de préférence sous la forme d'un sel, d'un oxyde ou d'un hydroxyde. De préférence le métal (les métaux) est (sont) sous la forme de sels (chlorure, nitrate, sulfate ou sel d'acide organique par exemple) ou sous la forme d'oxydes ou hydroxydes.

Le(s) composés(s) organo-silicié(s) est (sont) utilisé(s) en une concentration variant de $10^{-5}$ à $10^{-1}$ autome-gramme de silicium par litre et plus particulièrement de $10^{-2}$ à $10^{-4}$ atome-gramme/litre.

Les proportions de l'ajout de (2) : (1) sont comprises entre $10^{-10}$ et $10^{-5}$ atome-gramme de métal par litre de solution, mais on peut abaisser considérablement ces proportions en utilisant les éléments métalliques sous la forme sous laquelle ils sont présentés en homéopathie (de préférence 5 à 20 granules à la dilution définie en homéopathie, de 5 CH(CH = centésimale Hahnemannienne) dans un litre de solution organo-siliciée ; cette dilution peut cependant varier très largement, de 200 CH à 1 D (D = décimale Hahnemannienne), et la concentration peut être encore plus élevée comme mentionné ci-dessus).

En variante, la composition selon l'invention peut également comprendre, en une proportion appropriée pour neutraliser et éventuellement stabiliser ces composés, un ou plusieurs acides organiques (ou leurs sels, en particulier leurs sels alcalins), tels que par exemple l'acide salicylique, l'acide citrique, l'acide propionique, l'acide aspartique, l'acide glutarique, l'acide glutamique. Il y a toutefois lieu de noter que l'acide salicylique doit être proscrit pour le traitement de patients allergiques aux phénols et à l'acide salicylique en particulier et que, dans la mesure où ce cas peut être rencontré, il est alors tout à fait possible de remplacer cet acide par de l'acide citrique ou ses sels, comme il est montré plus loin dans les exemples.

L'acide organique ou son sel est utilisé en pratique en des concentrations variant de $10^{-5}$ à $10^{-1}$ molécule-gramme par litre ou plus, et particulièrement de $10^{-2}$ à $10^{-4}$ molécule-gramme par litre.

Dans tous les cas, il apparaît préférable que la solution ait un pH compris entre 3 et 7, ou mieux entre 3 et 5.

Les composés organo-siliciés correspondant à la formule susdite sont des produits connus en soi.

On peut utiliser pour la réalisation des compositions selon l'invention, soit ceux qui sont disponibles dans le commerce, soit leurs précurseurs connus, qui les engendrent par hydrolyse, soit leurs précurseurs connus, qui les engendrent par hydrolyse, tels que par exemple des silazanes ou alcoxysilanes correspondants (voir à ce sujet en particulier le document FR-A-2.230.376 au texte duquel il est expressément fait référence).

Les métaux peuvent être combinés avec les composés organo-siliciés par addition à la solution de ceux-ci soit à l'état solide, soit sous forme d'une solution aqueuse convenablement dosée, et cela, soit dès l'origine, soit en cours d'application et en particulier lors de l'utilisation à des fins thérapeutiques de la composition selon l'invention.

La composition selon l'invention peut donc consister soit en un produit unique contenant le composé organo-silicié et les métaux, soit en un produit bi-composants contenant séparément le composé organo-silicié et les métaux, dont le mélange peut alors être fait extemporanément.

La composition peut être mise à la disposition des utilisateurs dans des flacons ou dans des ampoules qui constituent des doses unitaires contenant une quantité de produit dosée pour chaque administration.

Les doses unitaires de composition totale préconisées pour chaque administration transcutanée sont avantageusement d'environ 200 $cm^3$.

Les compositions peuvent être complétées par des agents de neutralisation et/ou de conservation compatibles avec les principes actifs, tels que ceux indiqués plus haut, ainsi que par d'autres constituants tels que des colorants, des parfums et d'autres excipients, que l'homme du métier est apte à choisir et à incorporer selon ses connaissances en la matière, en fonction des besoins.

A partir de tests effectués sur l'homme, on a pu établir que l'apport de composés organo-siliciés associés à des métaux sous la forme d'application, sur la peau par exemple en compresses, de compositions conformes à l'invention, a une action transcutanée et exerce une action pharmacologiquement efficace pour le traitement de certains dérèglements de la mitose cellulaire et des maladies cardio-vasculaires.

Les résultats des essais montrent que les compositions conformes à l'invention sont particulièrement indiquées et exceptionnellement efficaces sans présenter de toxicité notable, pour le traitement ou la prévention de certains dérèglements de la mitose cellulaire et le traitement des maladies cardio-vasculaires.

A titre indicatif, on peut recommander comme posologie :

— L'application d'une compresse de coton (environ 5 cm sur 7 cm) imbibée de la composition selon l'invention au niveau de la zone douloureuse ou déficiente ou de toute autre partie du corps.

La compresse est recouverte d'une feuille de matière plastique, ou de toute autre matière susceptible de maintenir l'humidité. La compresse est appliquée pendant une durée comprise entre 8 et 12 heures par jour (au cours de la nuit par exemple).

— Un tamponnage de zone cutanée avec un tampon imprégné de la composition selon l'invention.

Ce tamponnage est effectué 2 à 4 fois par jour, par exemple sur les avant-bras, à l'endroit où les

vaisseaux sont les plus apparents. On a en effet pu établir que la composition selon l'invention traversait facilement l'épiderme.

— On a également pu appliquer simultanément les constituants de la composition selon l'invention par ionocinèse pratiquée localement au niveau (ou même en dehors) de la zone déficiente ou douloureuse.

L'appareil pour ionocinèse est constitué d'un générateur de courant continu (intensité de l'ordre de 10-15 milliampères), muni d'un dispositif permettant d'établir ou d'interrompre le courant d'une manière progressive et capable de maintenir une intensité constante pendant la durée de la séance.

L'intensité du courant est progressivement amenée à 10 mA et maintenue durant 20 minutes environ avant d'être abaissée progressivement, puis annulée.

Dans certains cas, on peut amener l'intensité du courant jusqu'à 25 mA, la durée de la séance pouvant aller jusqu'à 30 minutes.

Le générateur est relié à deux électrodes de carbone ou d'autres matériaux couramment utilisés lors des traitements par ionocinèse. La surface de l'électrode, bien que pouvant être très variable, est couramment de l'ordre de 200 $cm^2$. Chacune des deux électrodes est entourée de coton hydrophile que l'on imprègne de la composition selon l'invention.

L'électrode reliée au pôle négatif du générateur est appliquée sur une partie quelconque du corps, mais de préférence au niveau de la zone déficiente ou douloureuse : elle est maintenue au contact de la peau par un bandage. L'autre électrode, reliée au pôle positif du générateur, peut être appliquée à un endroit quelconque de la peau ou simplement tenue à la main, la surface de contact étant la plus élevée possible. Dans certains cas particuliers, on inverse la polarité des électrodes en cours de séance.

Il convient de signaler qu'un traitement nécessite 10 à 30 séances espacées de 24 à 72 heures, le rythme le plus couramment adopté étant de 2 à 3 séances par semaine. Le traitement est totalement indolore. Toutefois certains sujets sensibles ont une sensation de picotement au niveau des électrodes au cours des séances. Il n'a été observé aucune réaction locale ou générale de l'organisme, si ce n'est un léger érythème au niveau de la zone d'application des électrodes.

Cependant, lorsque le sujet présente une allergie aux dérivés phénoliques, on doit choisir une solution ne contenant pas ce type de dérivés.

— Des injections intramusculaires peuvent également être pratiquées.

L'invention est décrite plus en détail dans les exemples ci-après, qui ne la limitent aucunement.

## Exemple 1

Dans un bécher de 1 litre on a introduit 1 g d'acide salicylique et on y a ajouté 700 ml d'eau distillée.

On a versé lentement sur cette solution, en agitant énergiquement, 1,2 ml d'une solution de méthylsiliconate de potassium à 45 % d'extrait sec. On a ajouté ensuite 1 mg de nitrate d'uranyle. On a amené le pH de la solution à 4,7 par addition d'une solution aqueuse de potasse et on a complété à 1 litre avec de l'eau distillée.

## Exemple 2

Dans un bécher de 1 litre on a introduit 1 g d'acide citrique et on y a ajouté 700 ml d'eau distillée.

On a versé lentement sur cette solution, en agitant énergiquement, 1,2 ml d'une solution de méthylsiliconate de potassium à 45 % d'extrait sec. On a ajouté successivement 3 mg de nitrate de zirconium, 1 mg de nitrate d'uranyle, 1 mg de nitrate de hafnium et 3 mg de chlorure de titane.

On a ensuite amené le pH de la solution à 4,7 par addition d'une solution aqueuse de potasse ; on a complété à 1 litre avec de l'eau distillée et filtré la solution.

## Exemple 3

Dans un bécher contenant 500 ml d'eau distillée préalablement refroidie par addition de glace pilée, on a introduit lentement sous forte agitation 0,65 g de diméthyldichlorosilane et 0,85 g de bicarbonate de sodium.

On a vérifié que le pH était voisin de 7.

La solution ainsi obtenue étant homogène, on a ajouté lentement sous forte agitation 1,1 g de salicylate de sodium.

On a ajouté 2 mg de nitrate de zirconium.

Le pH de la solution a été amené à 4,7 par addition d'une solution aqueuse de soude.

On a complété ensuite à 1 litre avec de l'eau distillée.

## Exemple 4

On a introduit dans un bécher 1 g d'acide citrique et on a ajouté 700 ml d'eau distillée.

Sur cette solution, on a versé lentement, en agitant énergiquement, 1,7 ml une solution de méthylsiliconate de potassium à 45 % d'extrait sec.

On a ajouté 1 mg de nitrate d'uranyle.

Le pH de la solution a été amené à 4,7 par addition d'une solution aqueuse de potasse. On a complété ensuite à 1 000 ml avec de l'eau distillée.

Exemple 5

Dans un bécher de 1 litre, on a introduit 1 g d'acide salicylique et on y a ajouté 700 ml d'eau distillée.

On a versé lentement sur cette solution, en agitant énergiquement, 1,2 ml d'une solution de méthylsiliconate de potassium à 45 % d'extrait sec. On a ajouté alors 3 mg de nitrate de zirconium. On a amené le pH de la solution à 4,7 par addition d'une solution aqueuse de potasse et on a complété à 1 litre avec de l'eau distillée.

Pour illustrer maintenant les propriétés pharmacologiques des compositions comprenant en combinaison un composé organo-silicié tel que susdit et des métaux selon l'invention, on décrit ci-après quelques exemples d'essais pharmacologiques se rapportant au traitement chez l'homme de maladies cardio-vasculaires et de certains dérèglements de la mitose cellulaire (particulièrement le cancer).

Essai 1

ART... Béatrice présente un envahissement épithéliomateux du poumon gauche très étendu contrôlé par un examen radiologique et des tomographies.

Le traitement est réalisé de la manière suivante pendant deux mois à l'aide de la composition selon l'exemple 1 ci-dessus :

— compresse journalière pendant 8 heures ;
— tamponnement des avant-bras 4 fois par jour.

Un nouvel examen radiologique effectué deux mois et demi après permet de constater une importance régression des lésions et une nette amélioration clinique.

Le traitement est prolongé un mois de plus.

Une nouvelle radiographie de contrôle permet de constater une disparition des images pathologiques.

Trois ans après, la malade est toujours en bonne santé.

Essai 2

AUG... Henri présente une douleur permanente à la base gauche du thorax, s'accompagnant d'une gêne respiratoire et d'une toux sèche. L'examen clinique montre une rétraction de l'hémithorax gauche ; la radiographie montre une masse importante et irrégulière dans le poumon gauche et de nombreuses décalcifications circonscrites au niveau des côtes. Une biopsie costale met en évidence un envahissement par des formations épithéliales néoplasiques.

Le traitement est réalisé de la façon suivante pendant trois mois, à l'aide de la composition selon l'exemple 2 ci-dessus :

— compresse journalière pendant 8 heures ;
— tamponnement de l'avant-bras 4 fois par jour ;
— 25 séances d'ionocinèse.

Au bout de trois mois des radiographies sont effectuées qui montrent une fonte totale de la masse pulmonaire et une recalcification des côtes.

Le traitement est poursuivi pendant trois mois.

Au bout de trois ans l'état du patient est satisfaisant.

Essai 3

DAV... Lucien souffre depuis quelques années de crises précordiales douloureuses à la marche et à l'effort, pour lesquelles il est obligé de prendre de la trinitrine à raison de 3 à 6 dragées par jour.

Le traitement est réalisé de la manière suivante pendant 15 jours à l'aide de la composition suivant l'exemple 3 ci-dessus :

— compresse journalière pendant 8 heures ;
— tamponnement des avant-bras 3 fois par jour.

Au bout de 15 applications, on observe la disparition des crises douloureuses et le malade cesse de prendre de la trinitrine.

Le patient fait des applications locales d'entretien deux fois par semaine.

Au bout de trois mois l'état du malade est satisfaisant. L'électrocardiogramme est normal.

Essai 4

TRA... Raoul présente des douleurs épigastriques diffuses entrecoupées d'épisodes paroxystiques.

L'examen clinique montre un sub-ictère. L'intervention chirurgicale permet de constater un envahissement néoplasique du pancréas.

En raison de l'extension des lésions le chirurgien renonce à intervenir et referme.

Le traitement est réalisé de la manière suivante pendant un mois à l'aide de la composition selon l'exemple 4 ci-dessus :

— compresse journalière pendant 8 heures ;

— tamponnement des avant-bras 4 fois par jour.

Ce traitement appliqué permet de constater au bout d'un mois une disparition du sub-ictère et des douleurs.

Le traitement est poursuivi pendant plusieurs semaines.

Au bout d'un an, le malade est en bonne santé.

Essai 5

Monsieur DUR... Marcel a fait in infarctus du myocarde. L'électrocardiogramme montre des signes d'infarctus postérieur et il souffre d'une angine de poitrine résiduelle caractérisée par des crises douloureuses précordiales à la marche et à l'effort.

Le traitement est réalisé de la manière suivante pendant 2 mois à l'aide de la composition suivant l'exemple 5 ci-dessus :

— compresse journalière pendant 8 heures ;

— tamponnement des avant-bras 3 fois par jour ;

— 20 séances d'ionocinèse.

Après ce traitement, les signes d'ischémies disparaissent ; seuls persistent sur l'électrocardiogramme de discrets signes de nécrose postérieure.

Des applications d'entretien sont poursuivies à raison de 3 par semaine.

Au bout de six mois, l'électrocardiogramme est redevenu normal, l'état de santé du patient est satisfaisant.

## Revendications

1. Composition à usage thérapeutique, utile pour le traitement de dérèglements de la mitose cellulaire et des maladies cardio-vasculaires, caractérisée en ce qu'elle comprend en solution aqueuse :

(1) au moins un composé organo-silicié répondant à l'une des deux formules :

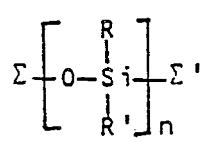

ou

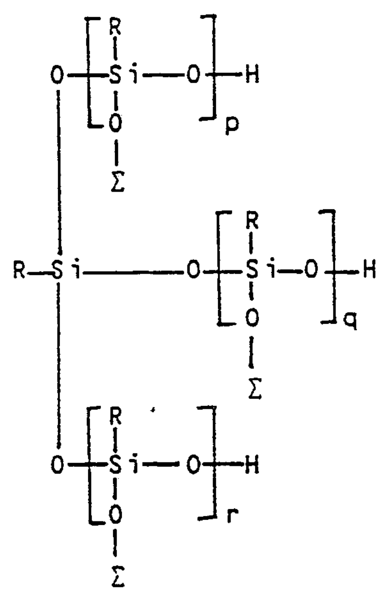

(A) (B)

dans lesquelles :

n est un nombre entier compris entre 1 et 1 000, et de préférence entre 1 et 50 ;

p, q, r sont des nombres entiers compris entre 0 et 1 000, et de préférence entre 0 et 60 ;

R et R' représentent, indépendamment l'un de l'autre, un groupement aliphatique linéaire, ramifié ou cyclique, saturé ou non, hétéro-linéaire ramifié ou non, cyclique saturé ou non, aromatique, hétéroaromatique, arylaliphatique ou hétéroarylaliphatique, pouvant être également fonctionnel tandis que R peut aussi être OH, OR ou $OSiR_3$ ;

Σ est R ou H ou $SiR_3$, où R est tel que précédemment défini,

Σ' est R et plus particulièrement $CH_3$, OR, OH ou $OSiR_3$, où R est tel que défini ci-dessus, et

(2) au moins un métal choisi parmi le titane, le zirconium, l'hafnium, le vanadium, le germanium, le chrome, le rhodium, l'or, l'iridium, le platine, l'osmium, les terres rares et les dérivés uranylés, de préférence sous la forme d'un sel, d'un oxyde ou d'un hydroxyde.

2. Composition selon la revendication 1, caractérisée en ce que le composé organo-silicié est à une concentration variant de $10^{-5}$ à $10^{-1}$ atome-gramme de silicium par litre et plus particulièrement de $10^{-4}$ à $10^{-2}$ atome-gramme par litre.

3. Composition selon la revendication 1, caractérisée en ce que le composé organo-silicié est choisi parmi les méthylsiliconates alcalins et le produit résultant de l'hydrolyse du diméthyldichlorosilane.

4. Composition.selon la revendication 1, caractérisée en ce que le rapport en poids/volume (2) : (1) est de $10^{-10}$ à $10^{-5}$ atome-gramme de métal par litre de solution.

5. Composition selon la revendication 1, caractérisée en ce que le rapport en poids/volume (2) : (1) est compris entre les valeurs de 200 CH et 1 D, définies en homéopathie.

6. Composition selon la revendication 1, caractérisée en ce qu'elle comprend en outre un ou plusieurs acides organiques ou leurs sels.

7. Composition selon la revendication 1, caractérisée en ce que les composés (B) sont neutralisés et éventuellement stabilisés par la présence d'un ou plusieurs acides organiques, ou leurs sels, notamment alcalins, tels que l'acide salicylique, l'acide citrique, l'acide propionique, l'acide aspartique, l'acide glutarique, l'acide glutamique ou leurs sels.

8. Composition selon l'une quelconque des revendications 1 à 7, caractérisée en ce qu'elle consiste en un produit unique contenant le composé organo-silicié et les métaux dans une seule solution.

9. Composition selon l'une quelconque des revendications 1 à 7, caractérisée en ce qu'elle consiste en un produit bi-composants contenant séparément le composé organo-silicié et les métaux.

10. Composition selon l'une quelconque des revendications 1 à 9, caractérisée en ce qu'elle est sous forme de doses unitaires, en flacons ou en ampoules, contenant environ 200 $cm^3$ de ladite composition.

## Claims

1. Composition for therapeutic use, particularly useful for the treatment of cellular mitosis disorders and cardio-vascular diseases, characterized in that it comprises in aqueous solution :

(1) at least one organo-silicon compound according to one of the two formulae :

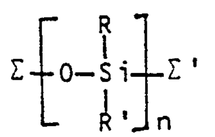

(A)

or

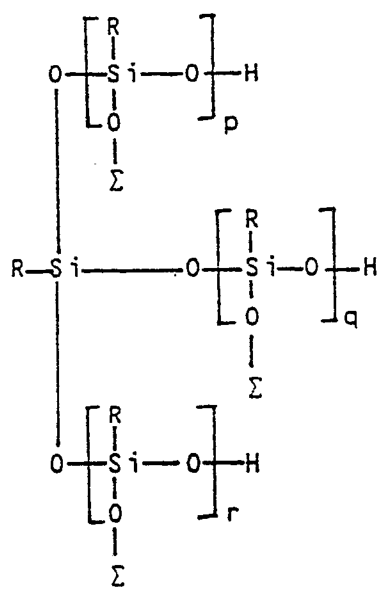

(B)

in which :

n is a whole number between 1 and 1 000, and preferably between 1 and 50 ;

p, q, r are whole numbers between 0 and 1 000 and preferably between 0 and 60 ;

R and R' represent independently of each other, a linear, branched or cyclic aliphatic compound which may, but need not be saturated, heterolinear, branched or unbranched, saturated or unsaturated cyclic, aromatic, hetero-atomic, arylaliphatic, or heteroarylaliphatic, and which may also be functional, whilst R may also be OH, OR or $OSiR_3$ ;

Σ is R, H or $SiR_3$, where R is as set out above ;

Σ' is R and in particular $CH_3$, OR, OH or $OSiR_3$ where R is as set out above, and

(2) at least one metal chosen from titanium, zirconium, hafnium, vanadium, germanium, chromium, rhodium, gold, iridium, platinum, osmium, rare earth elements and uranyl derivatives, preferably in the form of a salt, an oxide or a hydroxide.

2. Composition as claimed in claim 1, characterized in that the organo-silicon compound is in a concentration varying from $10^{-5}$ to $10^{-1}$ gram-atom of silicon per litre, an d preferably from $10^{-4}$ to $10^{-2}$ gram-atoms per litre.

3. Composition as claimed in claim 1, characterized in that the organo-silic compound is chosen from amongst the alkaline methyl siliconates and products of the hydrolysis of dimethyl dichlorosilane.

4. Composition as claimed in claim 1, characterized in that the weight/volume ration (2) : (1) is from $10^{-10}$ to $10^{-5}$ gram-atom of metal per litre of solution.

5. Composition as claimed in claim 1, characterized in that the weight/volume ration (2) : (1) is between the values of 200 CH and 1 D, as defined in homeopathy.

6. Composition as claimed in claim 1, characterized in that it also comprises one or more organic acids or their salts.

7. Composition as claimed in claim 1, characterized in that the compounds (B) are neutralized and if necessary, stabilized by the presence of one or more organic acids, or their salts, preferably alkaline, such as salicylic acid, citric acid, propionic acid, aspartic acid, glutaric acid, glutamic acid or their salts.

8. Composition as claimed in any of the claims 1 to 7, characterized in that it consists of a single product containing the organo-silicon compound and the metals in a single solution.

9. Composition as claimed in any of the claims 1 to 7, characterized in that it consists of a bicomponent product, in which the organo-silicon compound and the metal are separate.

10. Composition as claimed in any of the claims 1 to 9, characterized in that it is in the form of single doses, in vials or ampoules, containing approximately 200 $cm^3$ of the said mixture.

## Patentansprüche

1. Zusammensetzung zur therapeutischen Verwendung, nützlich zur Behandlung von Unregelmäßigkeiten der zellulären Mitose und Herzmuskelerkrankungen, dadurch gekennzeichnet, daß sie in wässriger Lösung enthält :

(1) wenigstens eine Organo-Silicium-Verbindung entsprechend einer der beiden Formeln :

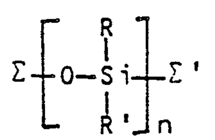

oder

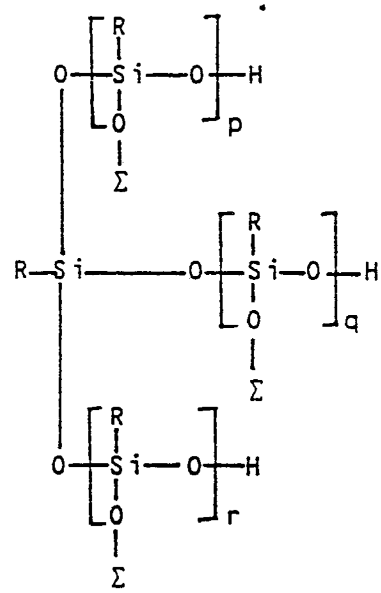

(A) (B)

in denen

n eine ganze Zahl zwischen 1 und 1 000 und vorzugsweise zwischen 1 und 50 ist ;

p, q, r ganze Zahlen zwischen 0 und 1 000 und vorzugsweise zwischen 0 und 60 sind :

R und R' unabhängig voneinander eine Gruppe darstellen, die linear, verzweigt oder cyclisch, gesättigt oder nicht, heterolinear verzweigt oder nicht, cyclisch gesättigt oder nicht, aromatisch, heteroaromatisch, arylaliphatisch oder heteroarylaliphatisch ist, die gleichzeitig funtionell sein kann, während R auch OH, OR oder $OSiR_3$ sein kann ;

Σ entweder R oder H oder $SiR_3$ ist, wobei R wie vorstehend definiert ist ;

Σ' entweder R und insbesondere $CH_3$, OR, OH oder $OSiR_3$ ist, wobei R wie oben definiert ist ; und

(2) wenigstens ein Metall ausgewählt aus Titan, Zirkonium, Hafnium, Vanadium, Germanium, Chrom, Rhodium, Gold, Iridium, Platin, Osmium, seltene Erden und den Uranylderivaten, vorzugsweise in Form eines Salzes, eines Oxids oder Hydroxids.

2. Zusammensetzung gemäß Anspruch 1, dadurch gekennzeichnet, daß die Organo-Silizium-Verbindung in einer Konzentration vorhanden ist, die von $10^{-5}$ bis $10^{-1}$ Atomgramm Silicium pro Liter und vorzugsweise von $10^{-4}$ bis $10^{-2}$ Atomgramm pro Liter variiert.

3. Zusammensetzung gemäß Anspruch 1, dadurch gekennzeichnet, daß die Organo-Silicium-Verbindung ausgewählt ist aus den alkalischen Methylsiliconaten und dem Produkt resultierend aus der Hydrolyse des Dimethyldichlorsilans.

4. Zusammensetzung gemäß Anspruch 1, dadurch gekennzeichnet, daß der Verhältnis von Gewicht/Volumen (2) : (1) von $10^{-10}$ bis $10^{-5}$ Atomgramm Metall pro Liter Lösung besteht.

5. Zusammensetzung gemäß Anspruch 1, dadurch gekennzeichnet, daß das Verhältnis Gewicht/Volumen (2) : (1) zwischen den Werten 200 CH und 1 D, definiert in der Homöopathie, liegt.

6. Zusammensetzung gemäß Anspruch 1, dadurch gekennzeichnet, daß sie zudem ein oder mehrere organische Säuren oder deren Salze enthält.

7. Zusammensetzung gemäß Anspruch 1, dadurch gekennzeichnet, daß die Verbindungen (B) neutralisiert und gegebenenfalls durch die Anwesenheit einer oder mehrerer organischer Säuren wie Salicylsäure, Citronensäure, Propionsäure, Asparaginsäure, Glutarsäure, Glutaminsäure oder deren Salze, insbesondere Alkalisalze, stabilisiert sind.

8. Zusammensetzung gemäß einem der Ansprüche 1 bis 7, dadurch gekennzeichnet, daß sie aus einem einheitlichen Produkt enthaltend die Organo-Silicium-Verbindung und die Metalle in einer einzigen Lösung besteht.

9. Zusammensetzung gemäß einem der Ansprüche 1 bis 7, dadurch gekennzeichnet, daß sie aus einem zweikomponentigen Produkt besteht, wobei die Komponenten getrennt die Organo-Silicium-Verbindung und die Metalle enthalten.

10. Zusammensetzung gemäß einem der Ansprüche 1 bis 9, dadurch gekennzeichnet, daß sie in Form von Einheitsdosen, in Flakons oder Ampullen, enthaltend etwa 200 $cm^3$ der besagten Zusammensetzung abgefüllt ist.